# EUROPEAN PATENT SPECIFICATION

(11) **EP 3 912 683 B1**
(45) Date of publication and mention of the grant of the patent: **19.03.2025**
(21) Application number: 20741537.3
(22) Date of filing: 08.01.2020
(51) Int. Cl.: A61Q 17/04, A61K 8/891, A61K 8/37, A61K 8/58, A61Q 1/02

(54) **SUNSCREEN COSMETIC COMPOSITION**
KOSMETISCHE SONNENSCHUTZZUSAMMENSETZUNG
COMPOSITION COSMÉTIQUE D'ÉCRAN SOLAIRE

(30) Priority: 15.01.2019 JP 2019004395
(43) Date of publication of application: 24.11.2021
(73) Proprietor: Shiseido Company, Ltd., Chuo-ku Tokyo 104-0061 (JP)
(72) Inventor: YAMAGUCHI Kazuhiro, Tokyo 104-0061 (JP); JEONG Jinu, Tokyo 104-0061 (JP); WATANABE Yurika, Tokyo 104-0061 (JP); ARAI Daiki, Tokyo 104-0061 (JP)
(74) Representative: Ter Meer Steinmeister & Partner
(86) International application number: PCT/JP2020/000287
(87) International publication number: WO 2020/149192

(56) References cited:
- WO-A1-2016/204947
- WO-A1-2018/062469
- JP-A- 2011 184 334
- JP-A- 2011 506 326
- JP-A- 2016 033 143
- JP-A- 2016 169 324
- JP-A- 2018 517 747
- JP-A- 2018 538 351
- US-A1- 2018 000 721

## Description

### Related Application

The present application claims the priority of Japanese Patent Application No. 2019-004395 filed on January 15, 2019.

### Technical Field

The present invention relates to a sunscreen cosmetic, more specifically, to a sunscreen cosmetic containing a salicylic acid-based liquid UV absorbent as a main UV absorbent and having an excellent UV protection effect.

### Background Art

Sunscreen cosmetics are cosmetics that are in high demand during the period of strong sunlight, especially in summer. Being products that do not sell well in winter, sunscreen cosmetics favorably contain liquid UV absorbents so that the quality of the products does not deteriorate by solidification and precipitation of the ingredients during the storage period in winter.

However, liquid UV absorbents are basically oils, and therefore stickiness occurs at the time of application when contained in a large amount. This is a serious problem for cosmetics used in summer.

Therefore, in order to reduce the content of liquid UV absorbents, ingredients with high UV absorbance per weight concentration are favorably used generally for sunscreen cosmetics. Typical examples of such ingredients include octyl methoxycinnamate that is a cinnamic acid-based UV absorbent. However, a further reduction in stickiness has been desired.

In order to further reduce stickiness, a salicylic acid-based UV absorbent having low viscosity and good feeling can be used. However, the salicylic acid-based UV absorbent is known to cause a reduction in emulsion stability because of its small molecular weight and high compatibility with a common emulsifier (that is, such UV absorbents dissolves emulsifiers). Further, since the salicylic acid-based UV absorbent has low UV absorbance per weight concentration (about 1/4 of that of octyl methoxycinnamate) and thus generally needs to be contained in an amount of as much as 15 to 20% by mass for obtaining a sufficient UV protection effect, the salicylic acid-based UV absorbent has not been used for sunscreens.

Alternatively, attempts to improve stickiness by adding a silicone oil that has excellent feeling have also been made. Examples thereof include ingredients such as volatile cyclopentasiloxane and low-molecular weight dimethicone, and non-volatile dimethicone.

Further, it is reported that the UV protection effect of a liquid UV absorbent is enhanced (boosted) by adding an alkyl-modified silicone (Patent Literature 1) that is solid at room temperature, which is known as a thickening ingredient of silicone oil, in the oil-in-water type emulsification system (Patent Literatures 2 and 3).

However, since the boost effect of the thickening ingredient is at most about 20% increase (rate of increase: 20%) (Patent Literature 4), it has been difficult to produce sunscreen cosmetics having an excellent UV protection effect without stickiness using a liquid UV absorbent with not-so-high UV absorbance per weight concentration.

### Citation List

### Patent Literature

[Patent Literature 1] Japanese Unexamined Patent Publication No. 2007-532754A
[Patent Literature 2] Japanese Unexamined Patent Publication No. 2005-145886A
[Patent Literature 3] International Publication No. WO 2018/062469
[Patent Literature 4] Japanese Unexamined Patent Publication No. 2003-212735A

### Summary of Invention

### Technical Problem

The present invention has been made in view of the problems of the conventional art, and an object thereof is to provide a sunscreen cosmetic having an excellent storage stability, while having a high UV protection effect without stickiness, using a liquid UV absorbent with not-so-high UV absorbance per weight concentration as a main UV absorbent. Solution to Problem

As a result of diligent studies in order to achieve the object, the inventors have found that, in an oil-in-water type emulsion, when 5 to 15% by mass of (a) a salicylic acid-based UV absorbent as a liquid UV absorbent is contained together with 0.5 to 3% by mass of (b) an alkyl silicone wax having a melting point of 60°C or higher, and (c) a low-viscosity silicone oil is further contained so that the ratio of ingredients (b) and (c) to the liquid oil is 20 to 60% by mass, the UV protection effect of the liquid UV absorbent is considerably boosted, and a sunscreen cosmetic having an excellent storage stability, while having a high UV protection effect without stickiness, is obtained, thereby accomplishing the present invention.

Further, the inventors have found that addition of 3 to 7% by mass of (d) octocrylene can produce a high UV protection effect that cannot be predicted from the total of the content of ingredients (a) and (d) (that is, an extraordinary boost effect).

That is, the present invention includes the following.
[1] A sunscreen cosmetic comprising:
   (a) 5 to 15% by mass of a salicylic acid-based liquid UV absorbent;
   (b) 0.5 to 3% by mass of an alkyl silicone wax having a melting point of 60°C or higher; and
   (c) a low-viscosity silicone oil,
   wherein
   a value obtained by dividing the total of contents of ingredients (b) and (c) by the content of liquid oils ({(b)+(c)}/content of liquid oils) is 0.2 to 0.6, which is an oil-in-water type emulsion.
[2] The sunscreen cosmetic according to [1], further comprising (d) 3 to 7% by mass of octocrylene.
[3] The sunscreen cosmetic according to any one of [1] or [2], wherein
   ingredient (a) is one or more liquid UV absorbents selected from homosalate and octyl salicylate.
[4] The sunscreen cosmetic according to any one of [1] to [3], wherein
   a value obtained by dividing the content of the (d) octocrylene by the content of liquid oils ((d)/content of liquid oils) is 0.2 to 0.5.
[5] The sunscreen cosmetic according to any one of [1] to [4], wherein
   ingredient (b) is alkyl methicone having one or more alkyl groups with 10 to 60 carbon atoms.
[6] The sunscreen cosmetic according to any one of [1] to [5], wherein
   ingredient (c) is one or more silicone oils selected from dimethicone, caprylyl methicone, and cyclopentasiloxane having a viscosity at 25°C of 100 mPa·s or less. Advantageous Effects of Invention

The present invention provides a sunscreen cosmetic containing a salicylic acid-based UV absorbent as a main UV absorbent and having an excellent storage stability, while having a high UV protection effect without stickiness.

### Brief Description of Drawings

[Figure 1] Figure 1 is a graph plotting the in-vitro SPF values of the sunscreen cosmetics of Experimental Examples 1-3 to 1-6.
[Figure 2] Figure 2 is a graph plotting the in-vitro SPF values of a series of sunscreen cosmetics in which the content of ingredient (d) is 2.5% by mass, 5% by mass, and 6.5% by mass in Tables 2 and 3.
[Figure 3] Figure 3 is a graph plotting the in-vitro SPF values (relative values to the control) of sunscreen cosmetics with an ingredient (b) replaced with various waxes.

### Description of Embodiments

Hereinafter, preferred embodiments according to the present invention will be described.

### (a) Salicylic acid-based liquid UV absorbent

The salicylic acid-based liquid UV absorbent that is be used in the present invention is not particularly limited, as long as it is generally used for cosmetics. Examples thereof include salicylic acid derivatives such as homosalate, octyl salicylate, amyl salicylate, menthyl salicylate, homomentyl salicylate, ethylhexyl salicylate, phenyl salicylate, benzyl salicylate, p-isopropanol phenyl salicylate, TEA salicylate, ethylene glycol salicylate, and dipropylene glycol salicylate. One of these salicylic acid-based liquid UV absorbents can be used alone, or two or more of them can be used in combination.

Among these, homosalate and octyl salicylate can be preferably used in particular.

The sunscreen cosmetic according to the present invention contains ingredient
(a) in an amount of 5 to 15% by mass, preferably 7 to 13% by mass, particularly preferably 9 to 12% by mass. When the amount is less than 5% by mass, a sufficient UV protection effect may not be obtained, whereas when the amount is more than 15% by mass, greasiness and stickiness may be felt.
(b) 0.5 to 3% by mass of alkyl silicone wax having a melting point of 60°C or higher

The alkyl silicone wax in the present invention is a silicone or a silicone resin modified by introducing an alkyl group. Examples thereof include alkyl dimethicone, alkoxy dimethicone, and alkyl methicone. The number of carbon atoms in the alkyl group is not specifically limited and is, for example, about 10 to 60, preferably about 20 to 50, most preferably about 25 to 45. It is not necessary that all alkyl groups to be introduced into the silicone or the silicone resin have the same number of carbon atoms, and at least one alkyl group preferably has the number of carbon atoms falling within such a preferable range. The alkyl group may be linear or branched.

Specific compound examples of the alkyl silicone wax having a melting point of 60°C or higher include alkyl (C30-45) methicone (for example, AMS-C30 Cosmetic Wax, available from Dow Corning Toray Co., Ltd., average melting point: 70°C), alkyl (C30-45) dimethylsilyl polypropylsilsesquioxane (for example, SW-8005 C30 Resin Wax, available from Dow Corning Toray Co., Ltd., average melting point: 66°C), alkyl (C30-45) dimethicone (for example, SF1642, available from Momentive Performance Materials Inc., average melting point: 60 to 70°C; and W3045, available from THOR Group Ltd., average melting point: 75 to 80°C), alkyl (C26-28) methicone (for example, CM7026VP, available from Wacker Asahikasei Silicone Co., Ltd., average melting point: 70°C), and alkyl (C26-28) methicone (for example, BELSIL CM 7026 VP, available from Wacker Asahikasei Silicone Co., Ltd., average melting point: 70°C). One of these alkyl silicone waxes can be used alone, or two or more of them can be used in combination.

Among these, alkyl (C30-45) methicone can be preferably used in the present invention.

The sunscreen cosmetic according to the present invention contains ingredient
(b) in an amount of 0.5 to 3% by mass, preferably 0.7 to 2.5% by mass, particularly preferably 1.0 to 2.0% by mass. When the amount is less than 0.5% by mass, the boost effect on the UV protection effect may not be sufficiently obtained, whereas when the amount is more than 3% by mass, the viscosity of the emulsion cosmetic becomes excessively high, and thus it may be difficult to spread the cosmetic.

### (c) Low-viscosity silicone oil

The low-viscosity silicone oil in the present invention is a silicone oil having a viscosity at 25°C of 100 mPa·s or less, preferably 50 mPa·s or less, further preferably 25 mPa·s or less. Examples of the silicone oil include linear dimethyl polysiloxane with a low degree of polymerization (dimethicone), methylphenylpolysiloxane, ethyl polysiloxane, ethyl methyl polysiloxane, ethyl phenyl polysiloxane, caprylyl methicone, dimethylsiloxane-methylphenylsiloxane copolymer, annular cyclopentasiloxane, octamethylcyclotetrasiloxane, and decamethylcyclopentasiloxane. One of these low-viscosity silicone oils can be used alone, or two or more of them can be used in combination.

Among these, dimethicone, caprylyl methicone, and cyclopentasiloxane can be preferably used in particular.

The content of ingredient (c) in the sunscreen cosmetic of the present invention is preferably 0.1 to 20% by mass or less, further preferably 0.5 to 9.0% by mass, particularly preferably 2 to 7% by mass. When the content is over 20% by mass, the stability of the emulsion cosmetic may deteriorate, and separation over time may occur.

Further, the total of the contents of ingredients (b) and (c) in the sunscreen cosmetic of the present invention is within the range of 0.2 to 0.6, more preferably 0.24 to 0.48, with respect to the total amount of liquid oils. When a value obtained by dividing the total of the contents of ingredients (b) and (c) by the total of the contents of liquid oils (that is, a value obtained by "{content of ingredient (b) + content of ingredient (c)}/content of liquid oils") is less than 0.2, there may be stickiness after application, whereas when the value is over 0.6, the stability of the emulsion cosmetic may deteriorate, and separation over time may occur.

The liquid oils in the present invention are fluid oils that do not volatilize at normal temperature (25°C). Examples thereof include hydrocarbon oils such as liquid paraffin, squalane, olefin oligomer, and light isoparaffin (= light liquid paraffin); ester oils such as glycerol tris(2-ethylhexanoate), cetyl 2-ethyl hexanoate, pentaerythritol 2-ethyl hexanoate, trimethylolpropane 2-ethyl hexanoate, 2-ethylhexyl palmitate, isocetyl isononanoate, isopropyl myristate, cetyl 2-ethyl hexanoate, diethyl sebacate, and diethyl adipate; natural vegetable oils such as jojoba oil, olive oil, macadamia nut oil, cottonseed oil, tea seed oil, safflower oil, and rice bran oil; and silicone oils such as decamethylpentacyclosiloxane, octamethyltetracyclosiloxane, dimethylpolysiloxane, and methylphenylpolysiloxane.

Oil-soluble ingredients that do not volatilize at normal temperature (25°C) are also regarded as the liquid oils in the present invention.

Therefore, ingredients (a), (c), and (d) according to the present invention are also included in the liquid oils.

### (d) Octocrylene

The sunscreen cosmetic of the present invention can contain the (d) octocrylene as a liquid UV absorbent together with ingredient (a).

The content of ingredient (d) in the present invention is preferably 3 to 7% by mass, more preferably 4 to 6% by mass. This is because, when the (d) octocrylene is added within such a range, a high UV protection effect that cannot be predicted from the total of the contents of ingredients (a) and (d) (that is, an extraordinary boost effect) can be obtained.

In the sunscreen cosmetic of the present invention, a value obtained by dividing the content of (d) octocrylene by the content of liquid oils (that is, a value obtained by " content of ingredient (d)/content of liquid oils") is preferably 0.2 to 0.5. When the ratio of ingredient (d) to liquid oils is lower than 0.2, the UVB protection effect may be felt to be relatively low, whereas when it is higher than 0.5, stickiness caused by octocrylene itself may be felt.

The liquid UV absorbent in the sunscreen cosmetic of the present invention may be composed only of ingredient (a) or ingredients (a) and (d), or may contain other liquid UV absorbents in addition to them, without inhibiting the effects of the present invention. Examples of the other UV absorbent include benzoic acid-based UV absorbents (for example, para-aminobenzoic acid (which will be hereinafter abbreviated as PABA), PABA monoglycerin ester, N,N-dipropoxy PABA ethyl ester, N,N-diethoxy PABA ethyl ester, N,N-dimethyl PABA ethyl ester, and N,N-dimethyl PABA butyl ester); anthranilic acid-based UV absorbents (for example, homomentyl-N-acetyl anthranilate); cinnamic acid-based UV absorbents (for example, octyl methoxycinnamate (ethylhexyl paramethoxycinnamate), ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, and glycerylmono-2-ethylhexanoyl-diparamethoxycinnamate); benzophenone-based UV absorbents (for example, 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methyl benzophenone, 2-hydroxy-4-methoxybenzophenone-5-sulfonate, 4-phenyl benzophenone, 2-ethylhexyl-4'-phenyl-benzophenone-2-carboxylate, 2-hydroxy-4-n-octoxybenzophenone, and 4-hydroxy-3-carboxybenzophenone); 3-(4'-methylbenzylidene)-d,l-camphor and 3-benzylidene-d,l-camphor; 2-phenyl-5-methylbenzoxazole; 2,2'-hydroxy-5-methyl phenylbenzotriazole; 2-(2'-hydroxy-5'-t-octylphenyl) benzotriazole; 2-(2'-hydroxy-5'-methylphenylbenzotriazole; dibenzalazine; dianisoylmethane; 4-methoxy-4'-t-butyl dibenzoylmethane; and 5-(3,3-dimethyl-2-norbornylidene)-3-pentane-2-one and dimorpholinopyridazinone.

The sunscreen cosmetic of the present invention may contain a powder that physically blocks ultraviolet light by reflection/scattering (UV scattering agent), in addition to such UV absorbents.

The UV scattering agent used in the present invention is not specifically limited, as long as it is a powder used as a UV scattering agent in the field of cosmetics. Specific examples thereof include one or two or more selected from titanium oxide, zinc oxide, barium sulfate, iron oxide, talc, mica, sericite, kaolin, titanium mica, iron blue, chromium oxide, chromium hydroxide, silica, and cerium oxide. In particular, powders having a refractive index of 1.5 or higher, such as zinc oxide and titanium oxide, are preferably used in view of optical properties.

The dispersibility in oil and the water resistance of the UV scattering agent can be improved by surface hydrophobization. In the present invention, a surface-hydrophobized UV scattering agent can be preferably used.

Examples of the surface treatment method include treatment with silicone, such as methylhydrogenpolysiloxane and methyl polysiloxane; treatment with alkylsilane; treatment with fluorine-containing compounds such as perfluoroalkyl phosphate ester and perfluoroalcohol; treatment with amino acids such as N-acylglutamic acid; other treatments, such as treatment with lecithins; treatment with metal soap; treatment with fatty acids; and treatment with alkyl phosphate esters.

The sunscreen cosmetic of the present invention may contain other optional ingredients that can be generally contained in sunscreen cosmetics, in addition to the essential ingredients, and other UV absorbents and UV scattering agents, without inhibiting the effects of the present invention.

Examples of the other optional ingredients include water-soluble polymers, oil-soluble polymers, solid oils such as waxes, lower alcohols, moisturizers, surfactants, powder ingredients, and pharmaceutical agents, but there is no limitation to these examples.

Examples of the water-soluble polymers include homopolymers or copolymers of 2-acrylamide-2-methyl propanesulfonic acid (which will be hereinafter abbreviated as "AMPS"). Examples of the copolymers include copolymers consisting of comonomers such as vinylpyrrolidone, acrylic acid amide, sodium acrylate, and hydroxyethyl acrylate. That is, examples thereof include AMPS homopolymer, vinylpyrrolidone/AMPS copolymer, dimethylacrylamide/AMPS copolymer, acrylic acid amide/AMPS copolymer, and sodium acrylate/AMPS copolymer.

Further, examples thereof include carboxyvinyl polymer, ammonium polyacrylate, sodium polyacrylate, sodium acrylate/alkyl acrylate/sodium methacrylate/alkyl methacrylate copolymer, carrageenan, pectin, mannan, curdlan, chondroitin sulfate, starch, glycogen, arabic gum, sodium hyaluronate, tragacanth gum, xanthan gum, mucoitinsulfuric acid, hydroxyethyl guar gum, carboxymethyl guar gum, guar gum, dextran, keratosulfate, locust bean gum, succinoglucan, chitin, chitosan, carboxy methyl chitin, and agar.

Examples of the oil-soluble polymers include trimethylsiloxysilicate, alkyl-modified silicone, polyamide-modified silicone, dimethicone crosspolymer, (dimethicone/vinyl dimethicone) crosspolymer, and polymethylsilsesquioxane.

Examples of waxes include beeswax, candelilla wax, carnauba wax, lanolin, liquid lanolin, and jojoba wax.

Examples of the lower alcohol include alcohols with 1 to 5 carbon atoms such as ethanol and isopropanol.

Examples of moisturizers include polyhydric alcohol such as ethylene glycol, propylene glycol, 1,3-butylene glycol, dipropylene glycol and polybutylene glycol.

Examples of the surfactants include anionic, cationic, nonionic, or amphoteric surfactants, including silicone-based or hydrocarbon-based surfactants.

Examples of the powder ingredients include nylon- or acrylic-based polymer spherical powders, silica powders, silicone powders, and metal oxide powders surface-treated with a metal-free surface-treating agent.

Examples of the pharmaceutical agents include salts of L-ascorbic acid and derivatives thereof, glycyrrhizic acid such as dipotassium glycyrrhizinate and monoammonium glycyrrhizinate and derivatives thereof, glycyrrhetinic acid such as stearyl glycyrrhetinate and derivatives thereof, allantoin, salts of tranexamic acid and derivatives thereof, salts of alkoxysalicylic acid and derivatives thereof, salts of glutathione and derivatives thereof, allantoin, and azulene.

Examples of the other ingredients include saccharides such as trehalose and erythritol, polysaccharides such as hyaluronic acid and acetylated hyaluronic acid, amino acids such as serine, glycine, hydroxy proline, glutamic acid, and alanine, and extracts such as silk extract, Chlorella extract, hydrolyzed pearl protein, Yaguruma (cornflower) extract, Tencha extract (Rubus Suavissimus), yeast extract, inositol, licorice extract, burnet extract, rose extract, and coix seed.

The sunscreen cosmetic of the present invention is in the form of an oil-in-water emulsion cosmetic

Specific examples of the dosage form include dosage forms such as a sunscreen milky lotion and a sunscreen cream, and a conventional method suitable for each dosage form can be used for the production.

### Examples

Hereinafter, the present invention will be described further in detail by way of specific examples, but the present invention is not limited to the following examples. All the cosmetics described in Tables 1 to 5 below were oil-in-water type cosmetics, and the content is expressed by "% by mass" unless otherwise specified.

First, the evaluation methods used for the present examples will be described.

### <Evaluation method>

### (1) in-vitro SPF

In order to objectively evaluate the UV protection effect of cosmetics, a high-accuracy in-vitro SPF measurement system described in Japanese Unexamined Patent Publication No. H7-167781A was used.

Each composition prepared was uniformly applied onto a simulated skin PMMA plate (SPFMASTER-PA01) described in Japanese Unexamined Patent Publication No. H7-167781A in an amount of 2.0 mg/cm² and left to stand for 15 minutes for drying. Then, the plate was irradiated with ultraviolet light using a solar simulator (Solar Ultraviolet Simulator Model 600: Solar Light Co.) as a light source. Then, the transmitted UV spectrum was subjected to arithmetic processing, to calculate an SPF value.

In the present invention, the SPF value was determined to be very high when the in-vitro SPF value was 30 or higher.

### (2) Stability

The state of each cosmetic one hour after the production was observed at room temperature, and those that no separation was observed were further stored at 40°C for 4 weeks. The stability thereof was evaluated according to the following criteria.
A: No separation was observed after storage at 40°C for 4 weeks.
B: Slight separation was observed after storage at 40°C for 4 weeks.
C: Remarkable separation was observed after storage at 40°C for 4 weeks.
D: Separation was observed one hour after the production (at room temperature).

In the present invention, the cosmetic was judged to be excellent in stability when the criterion was B or higher.

### (3) Non-stickiness

Each composition was applied to the face of a panel of 10 experts, and non-stickiness was evaluated.
A: 8 or more and 10 or less panelists answered there was no stickiness.
B: 6 or more and 7 or less panelists answered there was no stickiness.
C: 3 or more and 5 or less panelists answered there was no stickiness.
D: 2 or less panelists answered there was no stickiness.

In the present invention, the cosmetic was judged to be excellent in non-stickiness when the criterion was B or higher.

### Example 1: Study on UV absorbent

A sunscreen cosmetic with the formulation described in Table 1 was produced according to a conventional method, to measure a value of the in-vitro SPF according to the method. The results are shown in Table 1.

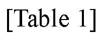

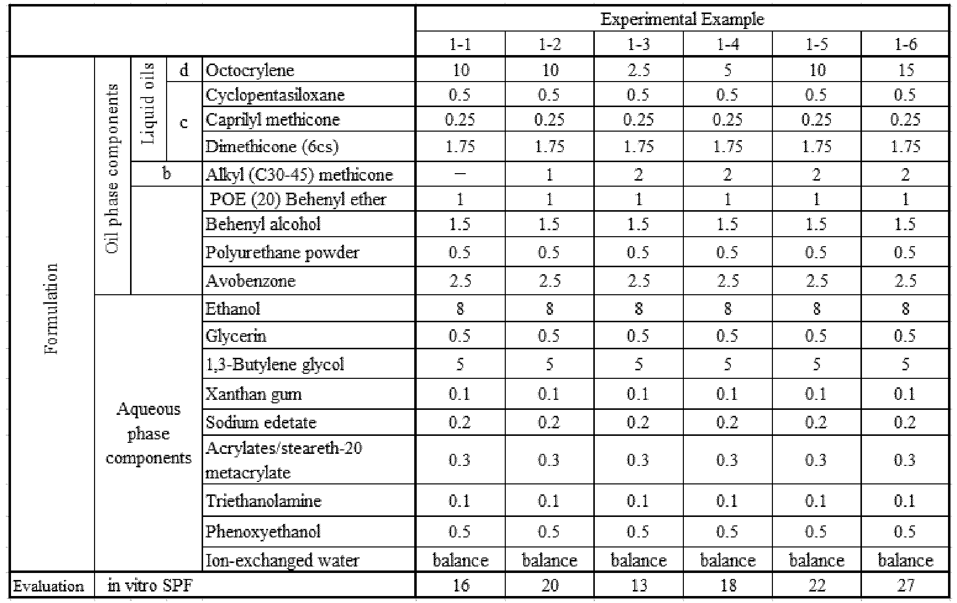

In Table 1, Experimental Examples 1-1, 1-2, and 1-5 were cosmetics having difference only in content of ingredient (b) with each other. As compared with the cosmetic that was free from ingredient (b) (Experimental Example 1-1), the in-vitro SPF value increased by 25% in the cosmetic containing 1% by mass of ingredient (b) (Experimental Example 1-2) and increased by about 37% in the cosmetic containing 2% by mass thereof (Experimental Example 1-5).

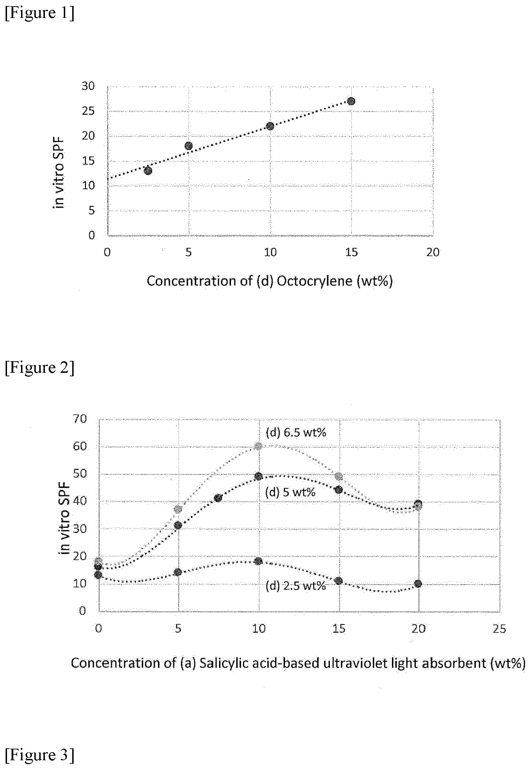

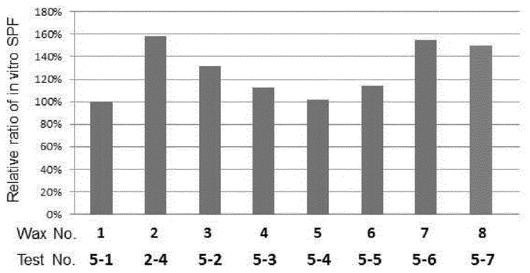

Experimental Examples 1-3 to 1-6 were cosmetics having differences only in content of (d) octocrylene with each other while the content of ingredient (b) was constant (2% by mass). Figure 1 shows the results of plotting the in-vitro SPF values of these experimental examples.

As shown in Figure 1, the UV protection effect almost linearly increased depending on the content of (d) octocrylene, in the presence of 2% by mass of ingredient (b).

Further, a sunscreen cosmetic containing (a) the salicylic acid-based UV absorbent and (d) octocrylene as liquid UV absorbents was produced and evaluated. Table 2 shows an experimental example with a concentration of ingredient (d) of 5% by mass or 6.5% by mass, and Table 3 shows an experimental example with that of 2.5% by mass or 10% by mass or greater.

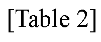

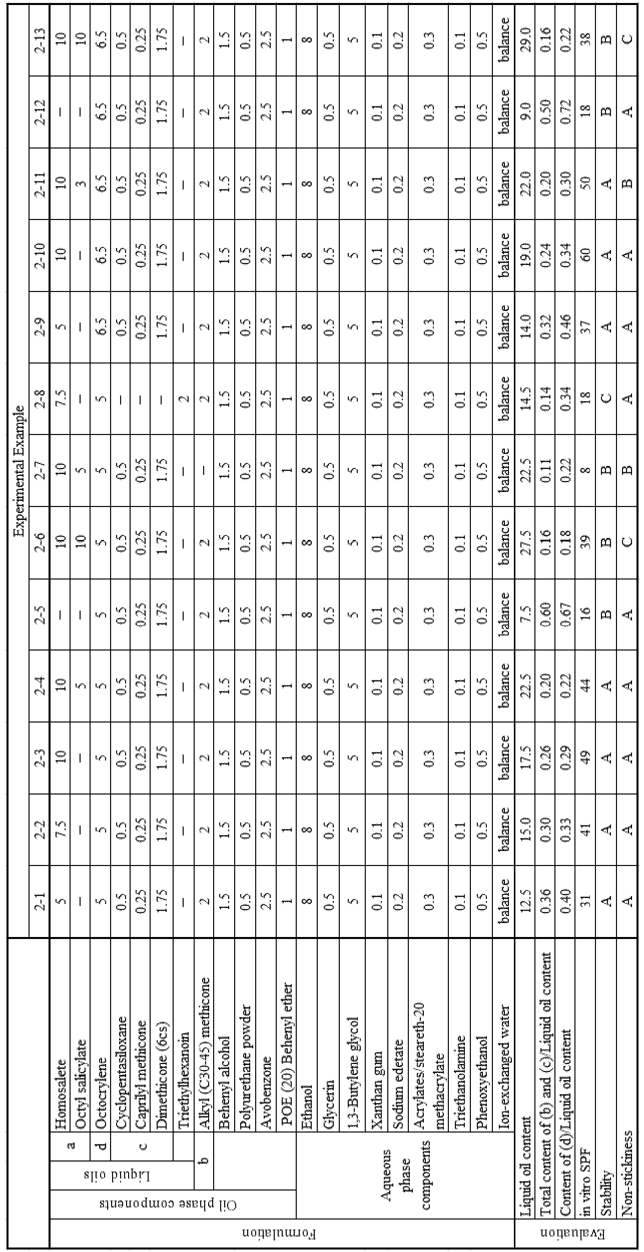

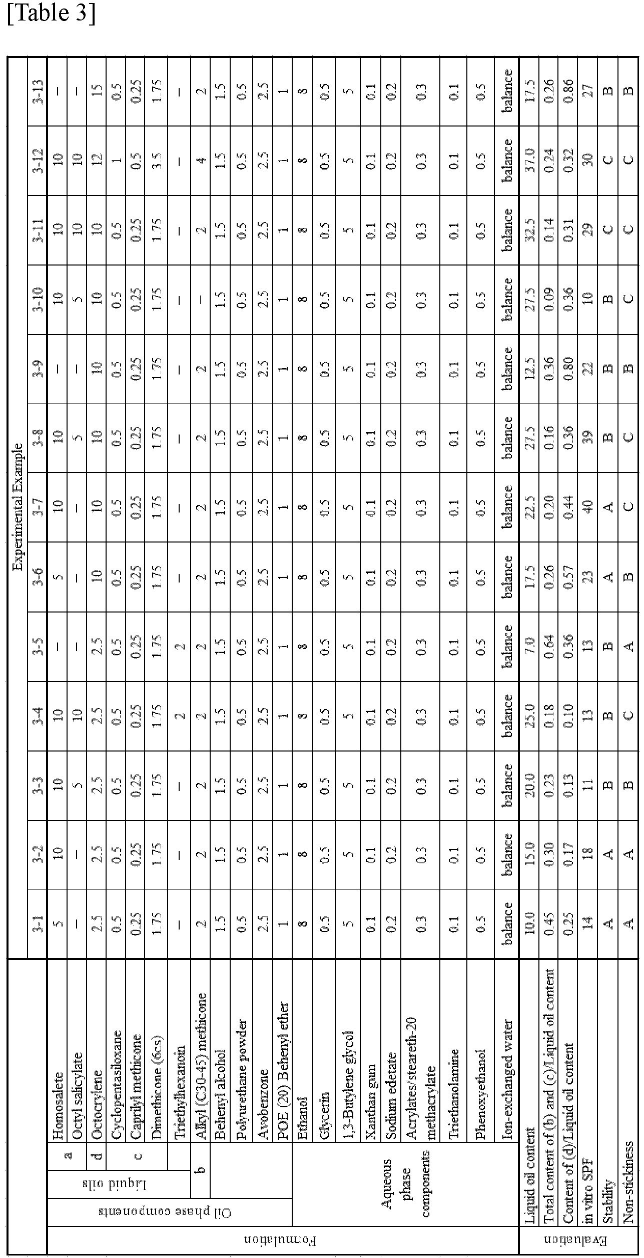

Figure 2 shows the results of plotting the values of in-vitro SPF for each content of ingredient (d) (2.5% by mass, 5% by mass, or 6.5% by mass) for the experimental examples in which the contents of ingredient (d) were the same and the contents of ingredient (a) varied in Tables 2 and 3.

As shown in Figure 2, the in-vitro SPF value increased with the increase of the content of ingredient (a) up to 10% by mass, regardless of the content of ingredient (d). However, the rate of increase was significantly different depending on the content of ingredient (d). That is, the in-vitro SPF value dramatically increased by addition of ingredient (a) in the cosmetic containing 5% by mass or 6.5% by mass of ingredient (d), whereas the rate of increase in in-vitro SPF value was very slight in the cosmetic containing only 2.5% by mass of ingredient (d), even when the same amount of ingredient (a) as in the cosmetic containing 5% by mass or 6.5% by mass of ingredient (d) was added.

For example, in the cosmetic series containing 6.5% by mass of ingredient (d), the maximum value "60" was obtained when the content of ingredient (a) was 10% by mass (Experimental Example 2-10). The in-vitro SPF value was extremely high as the effect of adding 10% by mass of (a) the salicylic acid-based liquid UV absorbent having a low absorbance per weight. It can be said that this is an extraordinary boost effect that far exceeds the boost effect on the UV absorption capacity that has been reported so far.

As shown in Table 2, in the cosmetics with the content of ingredient (d) of 5% by mass and the ratio of ingredients (b) and (c) to the liquid oils of 0.2 to 0.6, sunscreen cosmetics having a very high in-vitro SPF value and excellent stability without stickiness were obtained when the content of ingredient (a) was within the range of 5 to 15% by mass (Experimental Examples 2-1 to 2-4).

In contrast, in a cosmetic excluding only ingredient (a) from these cosmetics, the in-vitro SPF value considerably decreased (Experimental Example 2-5), and in a cosmetic with only the content of ingredient (a) increased to 20% by mass, strong stickiness occurred (Experimental Example 2-6). Further, in a cosmetic excluding only ingredient (b), the in-vitro SPF value considerably decreased (which decreased from 44 in Experimental Example 2-4 to 8 in Experimental Example 2-7). Further, in a cosmetic excluding ingredient (c) and containing triethylhexanoin instead as a liquid oil, the in-vitro SPF value considerably decreased (which decreased from 41 in Experimental Example 2-2 to 18 in Experimental Example 2-8), and the stability also significantly decreased (comparison between Experimental Examples 2-2 and 2-8).

Likewise, also in cosmetics with the content of ingredient (d) of 6.5% by mass and the ratio of ingredients (b) and (c) to the liquid oils of 0.2 to 0.6, sunscreen cosmetics having a very high in-vitro SPF value and excellent stability without stickiness were obtained when the content of ingredient (a) was within the range of 5 to 15% by mass (Experimental Examples 2-9 to 2-12).

Further, in a cosmetic excluding only ingredient (a) from these cosmetics, the in-vitro SPF value considerably decreased (Experimental Example 2-13), and in a cosmetic with only the content of ingredient (a) increased to 20% by mass, strong stickiness occurred (Experimental Example 2-14).

In contrast, in a cosmetic with the content of ingredient (d) of 2.5% by mass, a high in-vitro SPF value was not obtained when the content of ingredient (a) was within the range of 5 to 20% by mass, even if the ratio of ingredients (b) and (c) to the liquid oils was 0.2 to 0.6 (Experimental Examples 3-1 to 3-4). Further, in cosmetics with the content of ingredient (d) of 10% by mass or greater, cosmetics achieving all the effects of the present invention were not obtained (Experimental Examples 3-6 to 3-13).

Therefore, it was demonstrated that, when ingredients (a) and (d) were used as liquid UV absorbents at a constant ratio in combination, and the total of the contents of ingredients (b) and (c) in the liquid oils was 0.2 to 0.6, the UV protection effect synergistically increased, so that sunscreen cosmetics having a very high UV absorption capacity and excellent stability without stickiness were obtained. Further, it was suggested that, when the content of ingredient (a) was 5 to 15% by mass, the content of ingredient (b) was 0.5 to 3% by mass, and the content of ingredient (d) was 3 to 7% by mass, the effects were obtained.

Next, Table 4 shows the results of further containing octyl methoxycinnamate in addition to ingredients (a) and (d) as liquid UV absorbents.

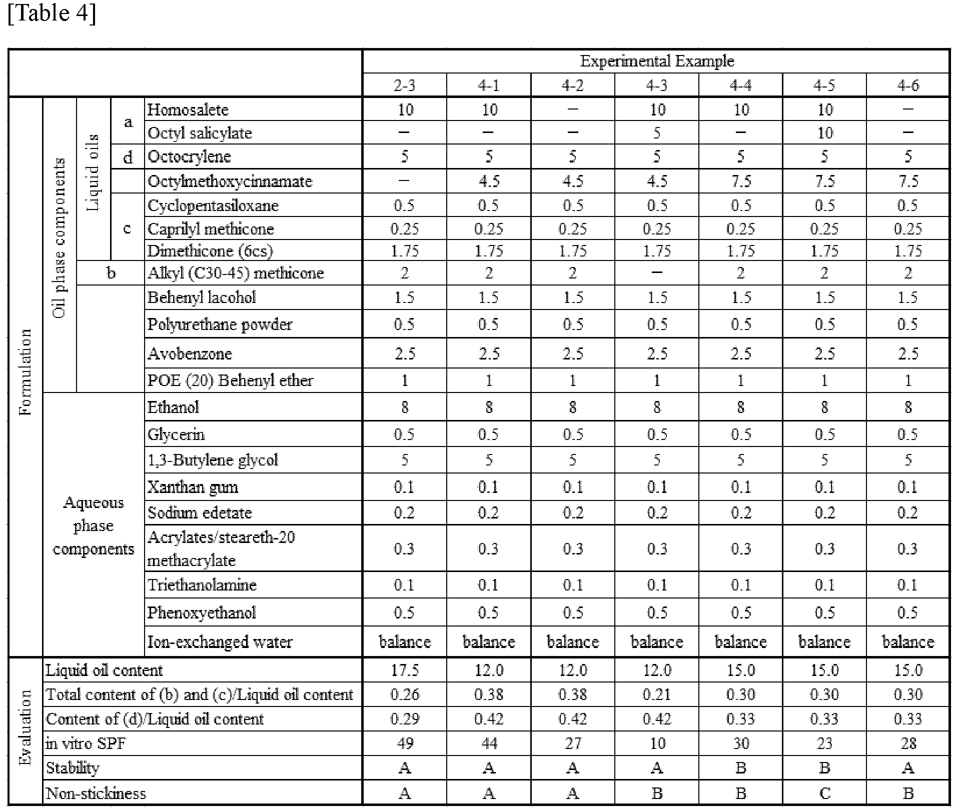

Experimental Examples 4-1 and 4-4 were cosmetics containing 4.5% by mass or 7.5% by mass of octyl methoxycinnamate in addition to the formulation of Experimental Example 2-3. The total amount of liquid UV absorbents was highest in Experimental Example 4-4, followed by 4-1, and lowest in 2-3, but the in-vitro SPF value (unexpectedly) resulted in the opposite order.

Further, in the cosmetic excluding only ingredient (a) from the formulation of Experimental Example 4-1, the in-vitro SPF value considerably decreased (Experimental Example 4-2), and in the cosmetic free from ingredient (b), the in-vitro SPF value decreased further considerably (Experimental Example 4-3). Further, in the cosmetic with only the content of ingredient (a) increased to 20% by mass from the formulation of Experimental Example 4-4, the in-vitro SPF value decreased, and strong stickiness occurred (Experimental Example 4-6).

Therefore, it was demonstrated that, when sunscreen cosmetics contain 5 to 15% by mass of ingredient (a) and 3 to 7% by mass of ingredient (d) as liquid UV absorbents, and the total of ingredients (b) and (c) in the liquid oils was 0.2 to 0.6, a very high UV absorption effect was obtained only by ingredients (a) and (d) without addition of octyl methoxycinnamate, and sunscreen cosmetics with excellent stability without stickiness were obtained.

### Example 2: Study on ingredient (b)

A sunscreen cosmetic with ingredient (b) replaced with any one of the following waxes in the formulation of Experimental Example 2-4 in Table 1 was produced according to a conventional method, and the in-vitro SPF value was measured according to the method. Wax 1 was a control, and wax 2 was ingredient (b) of Experimental Example 2-4.

### <Wax>

1. Liquid paraffin (Experimental Example 5-1)
2. Alkyl (C30-45) methicone (= Experimental Example 2-4)
   AMS-C30 Cosmetic Wax, available from Dow Corning Toray Co., Ltd.
   Average melting point: 70°C
3. Alkyl (C30-45) dimethylsilyl polypropylsilsesquioxane (Experimental Example 5-2) SW-8005 C30 Resin Wax, available from Dow Corning Toray Co., Ltd.
   Average melting point: 66°C
4. (Acrylates/behenyl acrylate/dimethicone methacrylate) copolymer (Experimental Example 5-3)
   KP562P, available from Shin-Etsu Chemical Co., Ltd.
   Average melting point: 50°C
5. Stearyl dimethicone (Experimental Example 5-4)
   SDM6022, available from Wacker Asahikasei Silicone Co., Ltd.
   Average melting point: 50°C
6. Alkyl (C-20-24) dimethicone (Experimental Example 5-5)
   W2024, available from THOR Group Ltd.
   Average melting point: 37°C
7. Alkyl (C30-45) dimethicone (Experimental Example 5-6)
   W3045, available from THOR Group Ltd.
   Average melting point: 75 to 80°C
8. Alkyl (C26-28) methicone (Experimental Example 5-7)
   CM7026VP, available from Wacker Asahikasei Silicone Co., Ltd.
   Average melting point: 70°C

Hereinafter, cosmetics produced by adding the above waxes 1 and 3 to 8 may be referred to as Experimental Examples 5-1 to 5-7.

Figure 3 shows a graph showing the in-vitro SPF value of each cosmetic as a relative value to the in-vitro SPF value of the cosmetic with ingredient (b) replaced with wax 1. As shown in Figure 3, the UV absorption capacity of the cosmetics increased by 120% or greater (Experimental Example 2-4: 158%, Experimental Example 5-2: 131%, Experimental Example 5-6: 155%, and Experimental Example 5-7: 150%) when alkyl silicone waxes 2, 3, 7, and 8 having an average melting point of 60°C or higher were used, as compared with the control. Further, it became evident that there were differences in the boost effect among silicone waxes having an alkyl group with 20 mol or more of hydrocarbons, and the average melting point was more preferably 65°C or higher, further preferably 70°C or higher.

Therefore, it was demonstrated that use of an alkyl silicone wax having a melting point of 60°C or higher, more preferably 65°C or higher, further preferably 70°C or higher as ingredient (b) according to the present invention allowed a remarkable boost effect on the UV absorption capacity to be obtained.

Examples of the formulation of the sunscreen cosmetic of the present invention are shown below. Needless to say, the present invention is not limited by these formulation examples at all. Each content is expressed by "% by mass" relative to the total amount of the sunscreen cosmetic.

### Formulation Example 1

| Component | Amount mixed (% by mass) |
|---|---|
| Ethanol | 8 |
| EDTA-3Na · H₂O | 0.1 |
| Sodium metabisulfite | 0.01 |
| Tranexamic acid | 1 |
| Sodium hyaluronate | 0.1 |
| Rosa roxburghii extract | 0.1 |
| (Acrylate/alkyl acrylate (C10-30)) crosspolymer | 0.1 |
| Carbomer | 0.05 |
| Agar | 0.1 |
| Xanthan gum | 0.1 |
| Glycerol | 3 |
| Butylene glycol | 3 |
| Potassium hydroxide | 0.06 |
| Ethylhexyl methoxycinnamate | 4.5 |
| Octocrylene | 5 |
| Diethylamino hydroxybenzoyl hexyl benzoate | 2 |
| Bisethylhexyloxyphenol methoxyphenyl triazine | 2 |
| Ethylhexyl salicylate | 7 |
| t-Butyl methoxydibenzoylmethane | 2 |
| C26-28 Alkyl methicone | 1.5 |
| Diisopropyl sebacate | 3 |
| Polypropylene glycol (17) | 1 |
| Caprylyl methicone | 5 |
| Dextrin (palmitate/ethylhexanoate) | 1 |
| Behenyl alcohol | 0.2 |
| Batyl alcohol | 0.2 |
| Tocopherol | 0.01 |
| Fragrance | q.s. |
| Spherical silica (average particle size 5 micron) | 5 |
| Ion-exchanged water | balance |
| Total | 100.00 |

### Formulation Example 2

| Component | Amount mixed (% by mass) |
|---|---|
| Ethanol | 8 |
| EDTA-2Na · 2H₂O | 0.05 |
| Sodium hexamethaphosphate | 0.05 |
| Citric acid | 0.03 |
| Sodium citrate | 0.01 |
| Tranexamic acid | 2 |
| L-ascorbyl magnesium phosphate | 0.5 |
| Sericin | 0.1 |
| Water-soluble collagen | 0.1 |
| Rosa roxburghii extract | 0.1 |
| (Dimethylacrylamide/sodium acryloyldimethyl taurate) copolymer | 0.3 |
| Succinoglycan | 0.1 |
| Stearoxyhydroxypropylmethylcellulose | 0.1 |
| Glycerol | 3 |
| Butylene glycol | 5 |
| Polyoxyethylene (14) polyoxypropylene (7) dimethylether | 1 |
| Polyoxyethylene hydrogenated castor oil (60 mol) | 1.5 |
| Octocrylene | 6.5 |
| Homosalate | 9.5 |
| Diethylamino hydroxybenzoyl hexyl benzoate | 1 |
| Bisethylhexyloxyphenol methoxyphenyl triazine | 1 |
| t-Butyl methoxydibenzoylmethane | 2.0 |
| C30-45 Alkyl methicone | 2.0 |
| Cyclopentasiloxane | 3 |
| Dimethicone 1.5cs | 1 |
| Dimethicone 6cs | 5 |
| Phytosteryl macadamiate | 1 |
| Di(phytosteryl/octyldodecyl)N-lauroyl-L-glutamate | 0.5 |
| Dextrin(palmitate/ethylhexanoate) | 1.5 |
| Sorbitan sesquiisostearate | 0.3 |
| Fragrance | q.s. |
| Silica-treated polyurethane ((HDI/trimethyloyl hexyllactone) crosspolymer) powder | 1 |
| Spherical silica (average particle size 5 micron) | 1 |
| Ion-exchanged water | balance |
| Total | 100.00 |

### Formulation Example 3

| Component | Amount mixed (% by mass) |
|---|---|
| Ethanol | 10 |
| Water-soluble collagen | 0.1 |
| Rosa roxburghii extract | 0.1 |
| Stearoxyhydroxypropylmethylcellulose | 0.3 |
| (Dimethylacrylamide/sodium acryloyldimethyl taurate) copolymer | 0.3 |
| Glycerol | 3 |
| Polyethylene glycol (molecular weight 400) | 5 |
| Bis-PEG-18 methyl ether dimethylsilane | 1 |
| Polyoxyethylene (14) polyoxypropylene (7) dimethylether | 1 |
| Ethylhexyl methoxycinnamate | 3 |
| Octocrylene | 4.5 |
| Octyl salicylate | 7 |
| Ethylhexyl triazine | 0.5 |
| Diethylamino hydroxybenzoyl hexyl benzoate | 1 |
| Bisethylhexyloxyphenol methoxyphenyl triazine | 1 |
| t-Butyl methoxydibenzoylmethane | 2.5 |
| C26-28 Alkyl methicone | 2.0 |
| Dimethicone (1cs) | 4 |
| Hydrogen dimethicone/aluminum hydroxide-treated titanium oxide fine particles | 0.5 |
| Octyltriethoxysilane-treated zinc oxide fine particles (particle size 25 nm) | 1.0 |
| Octyltriethoxysilane-treated titanium (pigment grade) | 1.0 |
| Caprylyl methicone | 2 |
| Polypropylene glycol (17) | 2 |
| Phytosteryl macadamiate | 1 |
| Di(phytosteryl/octyldodecyl)N-lauroyl-L-glutamate | 0.5 |
| Dextrin(palmitate/ethylhexanoate) | 0.5 |
| Fragrance | q.s. |
| Silica-treated polyurethane ((HDI/trimethyloyl hexyllactone) crosspolymer) powder | |
| | 1 |
| Spherical silica (average particle size 5 micron) | 1 |
| Ion-exchanged water | balance |
| Total | 100.00 |

### Formulation Example 4

| Component | Amount mixed (% by mass) |
|---|---|
| Ethanol | 10 |
| EDTA-2Na · 2H₂O | 0.05 |
| Dipotassium glycyrrhizate | 0.05 |
| Sodium acetylated hyaluronate | 0.1 |
| Water-soluble collagen | 0.1 |
| Green tea extract | 0.1 |
| Carbomer | 0.2 |
| (Acrylate/alkyl acrylate (C10-30)) crosspolymer | 0.1 |
| Xanthan gum | 0.1 |
| Agar | 0.1 |
| Glycerol | 3 |
| Butylene glycol | 5 |
| Polyoxyethylene (14) polyoxypropylene (7) dimethyl ether | 1 |
| Core-corona microgel emulsifier (acrylate/methoxy methacrylate PEG-90) crosspolymer (Production Example 9 of WO2013/094298) | 1.5 |
| 35% aqueous solution of betaine lauryldimethyl aminoacetate | 0.1 |
| Ethylhexyl methoxycinnamate | 1 |
| Octocrylene | 5 |
| Homosalate | 10 |
| Bisethylhexyloxyphenol methoxyphenyl triazine | 2 |
| t-Butyl methoxydibenzoylmethane | 1.5 |
| Octyltriethoxysilane-treated silica-treated titanium oxide | 1.5 |
| C30-45 Alkyl methicone | 1.6 |
| Dimethicone 1.5cs | 3 |
| Dimethicone 6cs | 2 |
| Caprylyl methicone | 0.4 |
| Diisopropyl sebacate | 3 |
| Alkyl benzoate (C12-15) | 3 |
| Phytosteryl macadamiate | 1 |
| Di(phytosteryl/octyldodecyl)N-lauroyl-L-glutamate | 0.5 |
| Dextrin(palmitate/ethylhexanoate) | 1 |
| Alumina-treated titanium oxide (pigment grade) | 1 |
| Fragrance | q.s. |
| Potassium hydroxide | 0.15 |
| Phenoxyethanol | 0.5 |
| Silica-treated polyurethane ((HDI/trimethyloyl hexyllactone) crosspolymer) powder | 1 |
| Spherical silica (average particle size 5 micron) | 1 |
| Ion-exchanged water | balance |
| Total | 100.00 |

### Formulation Example 5

| Component | Amount mixed (% by mass) |
|---|---|
| Ethanol | 5 |
| EDTA-2Na · 2H₂O | 0.05 |
| Sodium hexametaphosphate | 0.01 |
| Potassium 4-methoxysalicylate | 1 |
| Sodium hyaluronate | 0.1 |
| Glycylglycine | 0.1 |
| (Acrylate/alkyl acrylate (C10-30)) crosspolymer | 0.1 |
| Carbomer | 0.1 |
| (PEG-240/adecyltetradeceth-20/HDI) copolymer | 1 |
| Xanthan gum | 0.1 |
| Glycerol | 2 |
| Dipropylene glycol | 5 |
| PPG-8 ceteth-25 | 2 |
| Potassium hydroxide | 0.06 |
| Octocrylene | 5 |
| Homosalate | 10 |
| Bisethylhexyloxyphenol methoxyphenyl triazine | 2 |
| t-Butyl methoxydibenzoylmethane | 1.5 |
| Octyltriethoxysilane-treated silica-treated titanium oxide | 1.0 |
| C26-28 Alkyl methicone | 1 |
| Dimethicone 1.5cs | 1.3 |
| Polypropylene glycol (17) | 1 |
| Dimethicone 6cs | 5 |
| Dextrin(palmitate/ethylhexanoate) | 1 |
| Potassium hydroxide | 0.1 |
| Phenoxyethanol | 0.5 |
| Tocopherol | 0.01 |
| Fragrance | q.s. |
| Spherical cellulose powder | 5 |
| Ion-exchanged water | balance |
| Total | 100.00 |

### Formulation Example 6

| Component | Amount mixed (% by mass) |
|---|---|
| Ethanol | 10 |
| EDTA-2Na · 2H₂O | 0.05 |
| Sodium metabisulfite | 0.01 |
| Tranexamic acid | 2 |
| Menthol | 0.1 |
| Taurine | 0.1 |
| Glycylglycine | 0.1 |
| (Acrylate/alkyl acrylate (C10-30)) crosspolymer | 0.1 |
| Carbomer | 0.3 |
| Xanthan gum | 0.1 |
| Glycerol | 2 |
| Dipropylene glycol | 5 |
| PEG-60 glycerol isostearate | 1 |
| Cyclopentasiloxane solution of 50% PEG/PPG-19/19 dimethicone | 3 |
| Triethanolamine | 0.05 |
| Octocrylene | 6.5 |
| Octyl salicylate | 3 |
| Homosalate | 4 |
| Ethylhexyl triazine | 1 |
| Diethylamino hydroxybenzoyl hexyl benzoate | 1 |
| Bisethylhexyloxyphenol methoxyphenyl triazine | 1 |
| 50% aqueous dispersion of methylene bis-benzotriazolyl tetramethylbutylphenol | 1 |
| t-Butyl methoxydibenzoylmethane | 1.6 |
| C30-45 Alkyl methicone | 1.5 |
| Diisopropyl sebacate | 3 |
| Phytosteryl macadamiate | 1 |
| Polypropylene glycol (17) | 1 |
| Dimethicone 1.5cs | 2 |
| Dimethicone 6cs | 2 |
| Dextrin(palmitate/ethylhexanoate) | 1 |
| Phenoxyethanol | 0.5 |
| Tocopherol | 0.01 |
| Fragrance | q.s. |
| Spherical silica (average particle size 5 micron) | 1 |
| Talc | 3 |
| Ion-exchanged water | balance |
| Total | 100.00 |

### Formulation Example 7

| Component | Amount mixed (% by mass) |
|---|---|
| Ethanol | 5 |
| Sodium hexametaphosphate | 0.01 |
| EDTA-2Na · 2H₂O | 0.05 |
| 5% aqueous solution of polyquaternium-51 | 2 |
| Sodium acetylated hyaluronate | 0.1 |
| Taurine | 0.1 |
| Glycylglycine | 0.1 |
| Aqueous solution of (hydroxyethyl acrylate/ sodium acryloyldimethyl taurate) copolymer | 0.5 |
| Xanthan gum | 0.1 |
| Glycerol | 2 |
| Dipropylene glycol | 2 |
| Polyoxyethylene (14) polyoxypropylene (7) dimethyl ether | 1 |
| Trehalose | 1 |
| Stearic acid | 1 |
| Isostearic acid | 1.5 |
| Glyceryl stearate | 0.5 |
| PEG-30 phytosteryl | 0.3 |
| Behenyl alcohol | 0.5 |
| Batyl alcohol | 0.3 |
| Triethanolamine | 1 |
| Ethylhexyl methoxycinnamate | 1 |
| Octocrylene | 5 |
| Homosalate | 10 |
| Bisethylhexyloxyphenol methoxyphenyl triazine | 2 |
| t-Butyl methoxydibenzoylmethane | 1.7 |
| 50% aqueous dispersion of methylene bis-benzotriazolyl tetramethylbutylphenol | 1 |
| Phenylbenzimidazole sulfonic acid | 1.5 |
| C30-45 Alkyl methicone | 2 |
| Diisopropyl sebacate | 1 |
| Phytosteryl macadamiate | 1 |
| Polypropylene glycol (17) | 1 |
| Dimethicone 1.5cs | 3 |
| Dimethicone 6cs | 3 |
| Diphenylsiloxy phenyl trimethicone | 1 |
| Dextrin(palmitate/ethylhexanoate) | 1 |
| Phenoxyethanol | 0.5 |
| Tocopherol | 0.01 |
| Fragrance | q.s. |
| Spherical silica (average particle size 5 micron) | 1 |
| Ion-exchanged water | balance |
| Total | 100.00 |

### Formulation Example 8

| Component | Amount mixed (% by mass) |
|---|---|
| Ethanol | 5 |
| Citric acid | 0.02 |
| Sodium citrate | 0.08 |
| Sodium hexametaphosphate | 0.01 |
| Tranexamic acid | 2 |
| Sodium acetylated hyaluronate | 0.1 |
| Taurine | 0.1 |
| Glycylglycine | 0.1 |
| (Acrylate/alkyl acrylate (C10-30)) crosspolymer | 0.1 |
| Xanthan gum | 0.1 |
| Glycerol | 4 |
| Dipropylene glycol | 4 |
| Polyethylene glycol (molecular weight 1500) | 1 |
| Erythritol | 1 |
| Sodium methyl stearoyl taurate | 0.5 |
| Glyceryl stearate | 1 |
| Polyoxyethylene sorbitan monostearate (20 mol) | 1.5 |
| Behenyl alcohol | 3 |
| Stearyl alcohol | 0.5 |
| Triethanolamine | 2.5 |
| Octocrylene | 4.5 |
| Homosalate | 7.5 |
| Diethylamino hydroxybenzoyl hexyl benzoate | 1 |
| Bisethylhexyloxyphenol methoxyphenyl triazine | 1 |
| 50% aqueous dispersion of methylene bis-benzotriazolyl tetramethylbutylphenol | 1 |
| Phenylbenzimidazole sulfonic acid | 2 |
| t-Butyl methoxydibenzoylmethane | 2 |
| C30-45 Alkyl methicone | 1.5 |
| Polypropylene glycol (17) | 1 |
| Dimethicone 1.5cs | 5 |
| Dimethicone 6cs | 2 |
| Trimethyl pentaphenyl trisiloxane | 1 |
| Dextrin(palmitate/ethylhexanoate) | 1 |
| Phenoxyethanol | 0.5 |
| Tocopherol | 0.01 |
| Fragrance | q.s. |
| Spherical silica (average particle size 5 micron) | 1 |
| Ion-exchanged water | balance |
| Total | 100.00 |

### Formulation Example 9

| Component | Amount mixed (% by mass) |
|---|---|
| Ethanol | 5 |
| Sodium hexamethaphosphate | 0.01 |
| EDTA-2Na · 2H₂O | 0.05 |
| Silica fine particles | 0.2 |
| Dipotassium glycyrrhizinate | 0.05 |
| Sodium acetylated hyaluronate | 0.1 |
| Taurine | 0.1 |
| Glycylglycine | 0.1 |
| Carbomer | 0.1 |
| (Acrylate/alkyl acrylate (C10-30)) crosspolymer | 0.1 |
| Xanthan gum | 0.1 |
| Glycerol | 5 |
| Dipropylene glycol | 5 |
| Polyoxyethylene (14) polyoxypropylene (7) dimethyl ether | 1 |
| Trehalose | 1 |
| Polyoxyethylene behenyl ether (20 mol) | 1 |
| Stearyl alcohol | 1 |
| Behenyl alcohol | 2 |
| Batyl alcohol | 0.3 |
| Triethanolamine | 1.5 |
| Polysilicone-15 | 5 |
| Octocrylene | 4 |
| Ethylhexyl salicylate | 5 |
| Ethylhexyl triazone | 1 |
| Diethylamino hydroxybenzoyl hexyl benzoate | 2 |
| Bisethylhexyloxyphenol methoxyphenyl triazine | 2 |
| 50% aqueous dispersion of methylene bis-benzotriazolyl tetramethylbutylphenol | |
| | 1 |
| Phenylbenzimidazole sulfonic acid | 2 |
| C30-45 Alkyl methicone | 1 |
| Diisopropyl sebacate | 8 |
| Phytosteryl macadamiate | 1 |
| Polypropylene glycol (17) | 1 |
| Dimethicone 1.5cs | 3 |
| Dimethicone 6cs | 3 |
| Diphenylsiloxy phenyl trimethicone | 1 |
| Dextrin(palmitate/ethylhexanoate) | 1 |
| Phenoxyethanol | 0.5 |
| Tocopherol | 0.01 |
| Fragrance | q.s. |
| Cellulose powder (average particle size 5 µm) | 1 |
| Ion-exchanged water | balance |
| Total | 100.00 |

### Formulation Example 10

| Component | Amount mixed (% by mass) |
|---|---|
| Ethanol | 5 |
| Sodium hexametaphosphate | 0.01 |
| EDTA-2Na · 2H₂O | 0.05 |
| Nicotinamide | 5 |
| Sodium acetylated hyaluronate | 0.1 |
| Taurine | 0.1 |
| Sericin | 0.1 |
| 30% aqueous solution of (acrylates/steareth-20 methacrylate) copolymer | 1.5 |
| Xanthan gum | 0.2 |
| Glycerol | 2 |
| Dipropylene glycol | 5 |
| Polyoxyethylene (14) polyoxypropylene (7) dimethyl ether | 1 |
| Erythritol | 1 |
| Polyoxyethylene behenyl ether (20 mol) | 0.8 |
| Stearyl alcohol | 0.5 |
| Behenyl alcohol | 1.5 |
| PEG-10 dimethicone | 0.1 |
| Triethanolamine | 0.3 |
| Octocrylene | 5 |
| Homosalate | 4 |
| Ethylhexyl salicylate | 5 |
| Ethylhexyl triazone | 1 |
| Diethylamino hydroxybenzoyl hexyl benzoate | 1 |
| t-Butyl methoxydibenzoylmethane | 2.0 |
| C30-45 Alkyl methicone | 1.5 |
| Diisopropyl sebacate | 3 |
| Polypropylene glycol (17) | 1 |
| Dimethicone 1.5cs | 2 |
| Dimethicone 6cs | 2 |
| Diphenylsiloxy phenyl trimethicone | 1 |
| Dextrin(palmitate/ethylhexanoate) | 1 |
| Phenoxyethanol | 0.5 |
| Tocopherol | 0.01 |
| Fragrance | q.s. |
| Spherical silica (average particle size 5 micron) | 5 |
| Ion-exchanged water | balance |
| Total | 100.00 |

### Formulation Example 11

| Component | Amount mixed (% by mass) |
|---|---|
| Ethanol | 8 |
| EDTA-3Na · H₂O | 0.2 |
| Silica | 0.5 |
| Glycerol | 1 |
| Polyoxyethylene (14) polyoxypropylene (7) dimethyl ether | 1 |
| Rosa canina extract | 0.1 |
| Sodium hyaluronate | 0.1 |
| 2-O-Ethyl-L-ascorbic acid | 0.5 |
| Dipotassium glycyrrhizinate | 0.05 |
| Glyceryltri(2-ethylhexanoate) | 2 |
| Isopropyl myristate | 2 |
| Diisopropyl sabacate | 2 |
| PBG/PPG-9/1 copolymer | 1 |
| Dimethicone (6cs) | 3 |
| Dimethicone (2cs) | 2 |
| Trimethylsiloxysilicate | 0.5 |
| Sucrose tetrastearate triacetate | 0.5 |
| Dextrin palmitate | 1 |
| Polysilicone-15 | 3 |
| Ethylhexyl methoxycinnamate | 1 |
| Octocrylene | 7 |
| Homosalate | 5 |
| Octyl salicylate | 2 |
| Bisethylhexyloxyphenol methoxyphenyl triazine | 1 |
| t-Butyl methoxydibenzoylmethane | 2 |
| C30-45 Alkyl methicone | 1.5 |
| Methyl methacrylate crosspolymer | 5 |
| (Vinyl dimethicone/methicone silsesquioxane) crosspolymer | 5 |
| Dimethicone-treated talc | 1 |
| Cetyl PEG/PPG-10/1 dimethicone | 1 |
| Lauryl PEG-9 polydimethylsiloxyethyl dimethicone | 1 |
| Disteardimonium hectorite | 0.5 |
| Isostearic acid | 0.3 |
| Sorbitan sesquiisostearate | 0.3 |
| Tocopherol | 0.01 |
| Fragrance | q.s. |
| Ion-exchanged water | balance |
| Total | 100.00 |

A spray sunscreen was prepared by mixing 50% by mass of the stock solution with 50% by mass of LP gas to form an aerosol.

### Formulation Example 12 : BB cream

| Component | Amount mixed (% by mass) | |
|---|---|---|
| Ethanol | | 8 |
| EDTA-2Na · 2H₂O | | 0.05 |
| Sodium hexamethaphosphate | | 0.05 |
| Citric acid | | 0.03 |
| Sodium citrate | | 0.01 |
| L-ascorbyl magnesium phosphate | | 0.5 |
| Sodium acetylated hyaluronate | | 0.1 |
| Water-soluble collagen | | 0.1 |
| Rosa roxburghii extract | | 0.1 |
| (Dimethylacrylamide/sodium acryloyldimethyl taurate) copolymer | | 0.3 |
| Succinoglycan | | 0.2 |
| Cellulose gum | | 0.2 |
| Glycerol | | 2 |
| Butylene glycol | | 5 |
| Polyoxyethylene (14) polyoxypropylene (7) dimethyl ether | | 1 |
| Polyoxyethylene hydrogenated castor oil (100 mol) | | 1 |
| Polyoxyethylene (8 mol) behenyl ether | | 1 |
| Sodium methyl stearoyl taurate | | 0.1 |
| Stearyl alcohol | | 0.5 |
| Behenyl alcohol | | 0.5 |
| Octocrylene | | 7 |
| Octyl salicylate | | 8 |
| Diethylamino hydroxybenzoyl hexyl benzoate | | 1 |
| Bisethylhexyloxyphenol methoxyphenyl triazine | | 1 |
| C30-45 Alkyldimethysilyl polypropysilsesquioxane | | 2 |
| t-Butyl methoxydibenzoylmethane | | 2.5 |
| Caprylyl methicone | | 5 |
| Dimethicone 6cs | | 5 |
| Isododecane | | 10 |
| Di(phytosteryl/octyldodecyl)N-lauroyl-L-glutamate | | 0.5 |
| Dextrin(palmitate/ethylhexanoate) | | 0.5 |
| Octyltriethoxysilane-treated zinc oxide fine particles (particle size 25 nm) | | |
| | | 8 |
| Alumina-treated titanium oxide (pigment grade) | | 1.5 |
| Octyltriethoxysilane-treated red iron oxide | | q.s. |
| Octyltriethoxysilane-treated yellow iron oxide | | q.s. |
| Octyltriethoxysilane-treated black iron oxide | | q.s. |
| Isostearic acid | | 0.5 |
| Sorbitan sesquiisostearate | | 0.5 |
| Fragrance | | q.s. |
| Talc | | 3 |
| Spherical silica (average particle size 5 micron) | | 3 |
| Ion-exchanged water | | balance |
| Total | | 100.00 |

### Formulation Example 13

| Component | Amount mixed (% by mass) | |
|---|---|---|
| Ethanol | | 10 |
| EDTA-3Na · H₂O | | 0.1 |
| Salt | | 0.1 |
| Sodium metabisulfite | | 0.01 |
| Glycerol | | 1 |
| Xylitol | | 1 |
| Potentilla erecta root extract | | 1 |
| Sodium hyaluronate | | 0.1 |
| 2-O-Ethyl-L-ascorbic acid | | 0.1 |
| Dipotassium glycyrrhizate | | 0.05 |
| Isododecane | | 5 |
| Diisopropyl sebacate | | 5 |
| PBG/PPG-9/1 copolymer | 1 | |
| Dimethicone 1.5cs | 15 | |
| Dimethicone (6cs) | 3 | |
| Caprylyl methicone | 3 | |
| Dimethicone 20cs solution of 20% high polymerized aminopropylmethylsilozane | 1 | |
| Trifuloropropyldimethyl/trimethylsiloxysilicate 50% dimethicone solution | 3 | |
| Dextrin palmitate | 0.5 | |
| Octocrylene | 5 | |
| Homosalate | 5 | |
| Octyl salicylate | 1 | |
| Diethylamino hydroxybenzoyl hexyl benzoate | 1 | |
| Bisethylhexyloxyphenol methoxyphenyl triazine | 0.5 | |
| t-Butyl methoxydibenzoylmethane | 2.0 | |
| C30-45 Alkyl methicone | 2.0 | |
| Distearyldimethylammonium chloride-treated zinc oxide fine particles (Experimental Example 1 of WO 2006/064821) | 5.0 | |
| Octyltriethoxysilane-treated titanium oxide (pigment grade) | 1.9 | |
| Octyltriethoxysilane-treated red iron oxide | q.s. | |
| Octyltriethoxysilane-treated yellow iron oxide | q.s. | |
| Octyltriethoxysilane-treated black iron oxide | q.s. | |
| Methyl methacrylate crosspolymer | 5 | |
| (Vinyl dimethicone/methicone silsesquioxane) crosspolymer | 2 | |
| Dimethicone-treated talc | 3 | |
| PEG-9 Polydimethylsiloxyethyl dimethicone | 1.5 | |
| Cyclopentasiloxane solution of 50% PEG/PPG-19/19 dimethicone | 0.5 | |
| Disteardimonium hectorite | | 0.4 |
| Isostearic acid | | 0.3 |
| Tocopherol | | 0.01 |
| Fragrance | | q.s. |
| Ion-exchanged water | | balance |
| Total | | 100.00 |

## Claims

1. A sunscreen cosmetic comprising:
(a) 5 to 15% by mass of a salicylic acid-based liquid UV absorbent;
(b) 0.5 to 3% by mass of an alkyl silicone wax having a melting point of 60°C or higher; and
(c) a low-viscosity silicone oil having a viscosity at 25°C of 100mPa ·s or less, wherein a value obtained by dividing the total of contents of ingredients (b) and (c) by the content of liquid oils ({(b)+(c)}/content of liquid oils) is 0.2 to 0.6, and
wherein the sunscreen cosmetic is an oil-in-water type emulsion.

2. The sunscreen cosmetic according to claim 1 further comprising (d) 3 to 7% by mass of octocrylene.

3. The sunscreen cosmetic according to any one of claims 1 to 2, wherein
ingredient (a) is one or more liquid UV absorbents selected from homosalate and octyl salicylate.

4. The sunscreen cosmetic according to any one of claims 1 to 3, wherein
a value obtained by dividing the content of the (d) octocrylene by the content of liquid oils ((d)/content of liquid oils) is 0.2 to 0.5.

5. The sunscreen cosmetic according to any one of claims 1 to 4 wherein
ingredient (b) is alkyl methicone having one or more alkyl groups with 10 to 60 carbon atoms.

6. The sunscreen cosmetic according to any one of claims 1 to 5, wherein
ingredient (c) is one or more silicone oils selected from dimethicone, caprylyl methicone, and cyclopentasiloxane having a viscosity at 25°C of 100 mPa·s or less.

## Patentansprüche

1. Sonnenschutzkosmetikum, umfassend:
(a) 5 bis 15 Masse-% eines flüssigen UV-Absorptionsmittels auf Salicylsäurebasis;
(b) 0,5 bis 3 Masse-% eines Alkylsilikonwachses mit einem Schmelzpunkt von 60°C oder höher; und
(c) ein niedrigviskoses Silikonöl mit einer Viskosität bei 25°C von 100 mPa·s oder weniger,
wobei ein Wert, der durch Dividieren der Summe der Gehalte der Bestandteile (b) und (c) durch den Gehalt an flüssigen Ölen ({(b)+(c)}/Gehalt an flüssigen Ölen) erhalten wird, 0,2 bis 0,6 beträgt, und
wobei das Sonnenschutzkosmetikum eine Emulsion vom Öl-in-Wasser-Typ ist.

2. Sonnenschutzkosmetikum nach Anspruch 1, ferner umfassend (d) 3 bis 7 Masse-% Octocrylen.

3. Sonnenschutzkosmetikum nach einem der Ansprüche 1 bis 2, wobei Bestandteil (a) ein oder mehrere flüssige(s) UV-Absorptionsmittel ist, ausgewählt aus Homosalat und Octylsalicylat.

4. Sonnenschutzkosmetikum nach einem der Ansprüche 1 bis 3, wobei ein Wert, der durch Dividieren des Gehalts an (d) Octocrylen durch den Gehalt an flüssigen Ölen ((d)/Gehalt an flüssigen Ölen) erhalten wird, 0,2 bis 0,5 beträgt.

5. Sonnenschutzkosmetikum nach einem der Ansprüche 1 bis 4, wobei Bestandteil (b) Alkylmethicon mit einer oder mehreren Alkylgruppen mit 10 bis 60 Kohlenstoffatomen ist.

6. Sonnenschutzkosmetikum nach einem der Ansprüche 1 bis 5, wobei Bestandteil (c) ein oder mehrere Silikonöl(e), ausgewählt aus Dimethicon, Caprylylmethicon und Cyclopentasiloxan, mit einer Viskosität bei 25°C von 100 mPa·s oder weniger, ist.

## Revendications

1. Cosmétique de protection solaire comportant :
(a) 5 à 15 % en masse d'un absorbant d'UV liquide à base d'acide salicylique ;
(b) 0,5 à 3 % en masse d'une cire de silicone d'alkyle ayant un point de fusion de 60 °C ou plus élevé ; et
(c) une huile de silicone à faible viscosité ayant une viscosité à 25 °C de 100 mPa·s ou moins,
dans lequel une valeur obtenue en divisant le total des teneurs en ingrédients (b) et (c) par la teneur en huiles liquides ({(b)+(c)}/teneur en huiles liquides) est de 0,2 à 0,6, et
dans lequel le cosmétique pour écran solaire est une émulsion de type huile dans eau.

2. Cosmétique de protection solaire selon la revendication 1, comportant en outre (d) 3 à 7 % en masse d'octocrylène.

3. Cosmétique de protection solaire selon l'une quelconque des revendications 1 à 2, dans lequel :
l'ingrédient (a) est un ou plusieurs absorbants d'UV liquides choisis parmi l'homosalate et le salicylate d'octyle.

4. Cosmétique de protection solaire selon l'une quelconque des revendications 1 à 3, dans lequel :
une valeur obtenue en divisant la teneur en (d) octocrylène par la teneur en huiles liquides ((d)/teneur en huiles liquides) est de 0,2 à 0,5.

5. Cosmétique de protection solaire selon l'une quelconque des revendications 1 à 4, dans lequel :
l'ingrédient (b) est une méthicone d'alkyle possédant un ou plusieurs groupes alkyle ayant 10 à 60 atomes de carbone.

6. Cosmétique de protection solaire selon l'une quelconque des revendications 1 à 5, dans lequel :
l'ingrédient (c) est une ou plusieurs huiles de silicone choisies parmi une diméthicone, une méthicone de caprylyle et un cyclopentasiloxane ayant une viscosité à 25 °C de 100 mPa·s ou moins.
